# EUROPEAN PATENT APPLICATION

(11) **EP 4 785 807 A1**
(43) Date of publication of application: **05.08.2026**
(21) Application number: 25382073.2
(22) Date of filing: 04.02.2025
(51) Int. Cl.: A23K 20/105, A23K 20/111, A23K 50/75, A61K 31/05, A61K 31/192

(54) **OLIVE DERIVATES USEFUL FOR ENHANCING CONVERSION RATE IN POULTRY VIA IN OVO ADMINISTRATION**

(71) Applicant: Lucta, S.A., 28006 Madrid (ES)
(72) Inventor: BLANCH SABORIT, Marta, 28006 Madrid (ES); PASTOR PORRAS, José Javier, 28006 Madrid (ES); TEDÓ PÉREZ, Maria Gemma, 28006 Madrid (ES)
(74) Representative: ZBM Patents - Zea, Barlocci & Markvardsen

(57) **Abstract**

The present invention relates to a product which is either a compound selected from the group consisting of, at least a triterpene, and a mixture of at least a triterpene with hydroxytyrosol, or alternatively, an extract comprising at least a triterpene, or a mixture of at least a triterpene with hydroxytyrosol, for use in a method for enhancing conversion rate in poultry, where the method comprises administering the product *in ovo* between the days 7 to 21 of incubation. The product is also useful for reducing mortality rate of poultry, increasing the hatchability, and increasing the amount of first class chicks produced.

## Description

### Technical Field

This invention relates of hydroxytyrosol and triterpenes for use in enhancing feed conversion rate by their in ovo administration, as well as, for use additionally to reduce mortality, increase hatchability and/or increase first class chick production in poultry.

### Background Art

Animal husbandry often incorporates feed additives to improve growth rates, feed efficiency, and overall health of livestock. Natural compounds derived from plants have gained significant attention due to their potential benefits and reduced risk of adverse effects compared to synthetic additives.

International Patent Application WO2021198235 discloses the use of hydroxytyrosol (HT), maslinic acid (MA), and oleanolic acid (OA) as additives in animal feed for improving animal performance. Specifically, it discloses a reduction in mortality rates in poultry from birth to slaughterhouse and in pigs from lactation through post-weaning stages.

International Patent Application WO2007096446A1 describes the use of olive extract as a pronutrient in animal feed, obtained exclusively through physical methods. The extract contains, as majority components, maslinic acid, oleanolic acid, hydroxytyrosol, tyrosol, and their natural derivatives found in olives or their biologically acceptable salts. The use of this olive extract is claimed to provide significant improvements in feed efficiency and other feeding parameters in birds and pigs.

While the prior art references demonstrate the potential benefits of olive-derived compounds in animal feed, there remains a need to further enhance the health and performance of domesticated animals. Specifically, improvements in feed intake, growth rates, and protection against intestinal diseases continue to be areas of interest in the field of animal performance. Additionally, interventions that facilitate health and resilience improvements in very young animals are particularly relevant, as their immune and digestive systems are not yet fully developed, making them especially vulnerable.

The poultry industry continuously seeks innovative strategies to enhance the health, performance, and overall well-being of broiler chickens. One such strategy that has garnered considerable attention is the *in ovo* administration of bioactive substances. This method involves injecting bioactive compounds directly into the egg before hatching, aiming to positively influence the development of the gastrointestinal tract, boost mucosal immunity, and establish a favorable microbiota profile in newly hatched chicks.

Studies like Ayalew et al. have investigated the *in ovo* delivery of prebiotics, probiotics, and symbiotics. However, results have been inconsistent due to challenges in determining optimal dosages, injection timing, placement sites, and selecting appropriate bioactive substances. Negative effects on hatchability and chick performance have been reported, highlighting the need for further research.

Niknafs et al. examined the *in ovo* injection of oregano essential oil (OEO). Their findings indicated that higher doses (10 µL or more) reduced hatchability and chick quality, while a lower dose (5 µL) avoided these issues and activated genes related to detoxification and lipid metabolism.

Despite these efforts, there remains a need for improved *in ovo* methods and compositions that consistently enhance chick development without adverse effects. It is therefore desirable to provide an improvement to poultry production while avoiding the prevalent issues known in the art.

### Summary of Invention

In search of performance increase and optimization of avian production, inventors have found that by in ovo administering at least a triterpene, and a mixture of at least a triterpene with hydroxytyrosol, or alternatively, an extract comprising at least a triterpene, or a mixture of at least a triterpene with hydroxytyrosol, are able to enhance feed conversion rate and reduce mortality in poultry. Surprisingly, the results show that with a single administration, the feed conversion rate of the poultry is improved compared to administering the same compounds as a feed additive during the entire productive cycle of the animal. This is evidenced, for example, when comparing it with WO2021198235. Additionally, the hatchability and the amount of first class chicks is increased compared to control samples.

Another inherent challenge in using maslinic acid for in ovo injections of aqueous solutions is the difficulty of achieving solubility, especially considering the required concentrations and the limited volume that can be safely injected into an egg. These challenges have been effectively addressed by the invention disclosed herein.

Thus, an aspect of the present invention relates to a product which is either a compound selected from the group consisting of, at least a triterpene, and a mixture of at least a triterpene with hydroxytyrosol, or alternatively, an extract comprising at least a triterpene, or a mixture of at least a triterpene with hydroxytyrosol, for use in a method for enhancing conversion rate in poultry, wherein the method comprises administering the product *in ovo* between the days 7 to 21 of incubation.

### Detailed description of the invention

As used herein, the indefinite articles "a" and "and" are synonymous with "at least one" or "one or more". Unless stated otherwise, the definite articles used herein, such as "the" also include the plural of the noun.

The term "poultry" encompasses domesticated avian species specifically bred and raised for the production of meat, eggs, feathers, or other commercially valuable products. This includes, but is not limited to, broilers, turkey, duck, geese, pigeons, quail, pheasant, and ostrich. These poultry are reared in controlled environments optimized for health, growth rates, and product quality, utilizing standardized housing, feeding, and health management protocols to ensure consistent and efficient production outcomes

The term "broiler" refers to chickens specifically bred and raised for meat production. These chickens are selected for their rapid growth rates, efficient feed conversion, and tender meat. Broilers are disclosed herein and are reared in controlled environments that optimize their health and growth to reach market weight quickly, usually within 5 to 7 weeks.

The term "first class chick" refers to a chick that meets high-quality standards in terms of health, physical condition, and development. These chicks are usually free from any deformities, have strong legs and beaks, bright eyes, good weight, and active behavior, all indicating good vitality and robustness.

The term "feed" refers to the food provided to poultry, such as chicken, turkey, duck, geese, pigeons, quail, pheasant, and ostrich, to ensure their growth, health, and productivity. Poultry feed typically consists of grains, protein sources, vitamins, minerals, and sometimes other additives to balance the diet.

The terms "conversion rate" and "feed conversion rate" have been used herein indistinctly to refer to a measure of an animal's efficiency in converting feed into body mass. It is calculated as the amount of feed required to produce a certain amount of weight gain and is typically expressed as a ratio, such as kilograms of feed per kilogram of weight gained. In poultry, a lower feed conversion rate indicates better feed efficiency, meaning less feed is needed for the birds to gain weight, which is economically favorable.

The term "mortality" refers to the number of observed deaths in a group in relation to the total initial number of individuals in the group.

The term "hatchability" refers to the ability of eggs to successfully develop and produce viable offspring through the process of hatching. It is expressed as a percentage, indicating the proportion of fertilized eggs that successfully hatch under specific conditions.

The expression "effective amount" as used herein when relating to the ingredients, for instance, hydroxytyrosol, and/or triterpenes such as maslinic acid, and oleanolic acid, refers to the amount of the compounds that, when administered, is sufficient to promote daily weight gain of interest and intestine maturity and nutrient absorption and feed intake in young, domesticated animals. The particular dose of compounds administered according to this invention will be determined by the particular circumstances surrounding the case, including the compound administered, the route of administration, the particular condition being treated, and the similar consideration.

The expression "acceptable carriers" refers to compounds suitable for *in ovo* administration in poultry. Each compound must be acceptable in the sense of being compatible with other ingredients of the extracts and compositions.

The term "room temperature" as disclosed herein refers to a temperature of the environment, without heating or cooling, and is generally comprised of from 20 to 25 °C.

As mentioned above, it is part of the present invention a product which is either a compound selected from the group consisting of at least a triterpene, and a mixture of at least a triterpene with hydroxytyrosol, or alternatively, an extract comprising at least a triterpene, or a mixture of at least a triterpene with hydroxytyrosol, for use in a method for enhancing conversion rate in poultry, wherein the method comprises administering the product *in ovo* between the days 7 to 21 of incubation.

Any embodiments or claims described herein as a "product for use in" a particular process or application are also intended to encompass and disclose the "use of" the product in that process or application. This includes any and all uses of the product together with other compositions or in specific methods as applicable. Accordingly, all "for use" embodiments are to be interpreted and may be reformulated in the "use of" format.

Triterpenes are a class of terpenes composed of six isoprene units with the molecular formula C₃₀H₄₈.

According to the literature, as a way of example, the technology used for the extraction of triterpene acids from residual olive pomace has been extraction with methanol/ethanol (1:1, v/v) for 5 min and centrifugation, Soxhlet extraction up to 60 min and 75 °C with ethyl acetate or methanol and microwave-assisted extraction (MAE) for 10 min at 85 °C with ethyl acetate or methanol.

In a particular embodiment, in combination with any of the embodiments above or below, the product for use as defined above, wherein the triterpenes are pentacyclic triterpenes. In a particular embodiment, in combination with any of the embodiments above or below, the product for use as defined above, wherein the triterpenes are triterpenic acids. In another particular embodiment, the product for use as defined above, wherein the triterpene is selected from the group consisting of maslinic acid, oleanolic acid, and a mixture thereof.

Maslinic acid, also known as 2α,3β-Dihydroxyolean-12-en-28-oic acid , is a compound derived from dry olive-pomace oil (an olive skin wax) which is a byproduct of olive oil extraction. It is a member of the group of triterpenes known as oleananes. It has the following formula:

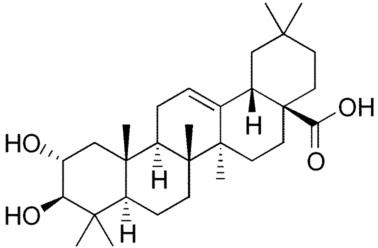

Oleanolic acid or oleanic acid, also named 3β-Hydroxyolean-12-en-28-oic acid, is a naturally occurring pentacyclic triterpenoid related to betulinic acid and has the following formula:

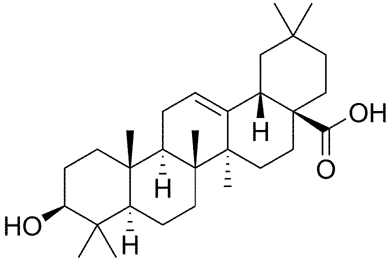

Oleanolic acid can be found in olive oil, *Phytolacca americana* (American pokeweed), and *Syzygium* spp, garlic, etc. It was first studied and isolated from several plants, including *Olea europaea* (leaves, fruit), *Rosa woodsii* (leaves), *Prosopis glandulosa* (leaves and twigs), *Phoradendron juniperinum* (whole plant), *Syzygium claviflorum* (leaves), *Hyptis capitata* (whole plant), *Mirabilis jalapa*^{]} and *Ternstroemia gymnanthera* (aerial part). Other *Syzygium* species including java apple (*Syzygium samarangense*) and rose apples contain it, as does *Ocimum tenuiflorum* (holy basil).

Oleanolic acid may also be obtained for instance by biosynthesis. As a way of example, Oleanolic acid biosynthesis may start with mevalonate to create squalene. Squalene monooxygenase in the next step oxidases the squalene and forms an epoxide resulting in 2,3-oxidosqualene. Beta-amyrin synthase creates beta-amyrin by a ring formation cascade. After the formation of beta amyrin, CYP716AATR2, also known as a cytochrome p450 enzyme, oxidizes carbon 28 turning it into alcohol. CYP716AATR2 converts the alcohol to aldehyde and finally to a carboxylic acid forming oleanolic acid (see Fukushima EO et al.; (December 2011). "CYP716A subfamily members are multifunctional oxidases in triterpenoid biosynthesis". 2011, Plant & Cell Physiology. Vol. 52, pp. 2050-2061; or Dale MP et al.; "A systematic comparison of triterpenoid biosynthetic enzymes for the production of oleanolic acid in Saccharomyces cerevisiae". PLOS ONE. Vol. 15 (5): e0231980.

In a particular embodiment, the triterpenes are extracted from olive oil. In a more particular embodiment, the triterpenes are extracted from *Olea europaea,* in particular, oleanolic acid and maslinic acid are extracted from *Olea europaea.* As a way of example, an olive oil extract comprising such triterpenes can be obtained from oxidized black olives, by centrifugation, acidification at an acidic pH (around 2.5-3) and dried, followed by extraction with ethanol, centrifugation, addition of water, removal of the remaining ethanol, and lyophilization to obtain a powder. The extract may be use in liquid form, in particular in the form of aqueous extracts, or in solid form.

In a particular embodiment, the olive oil extract (*olea europaea*) has a triterpene content equal to or higher than 60% by weight. In another particular embodiment, the olive oil extract (*olea europaea*) has a triterpene content equal to or higher than 70% by weight. In another particular embodiment, the content of oleanolic acid and maslinic acid in the extract is equal to or higher than 70% by weight of the total triterpenic acids. In another particular embodiment, the content of oleanolic acid and maslinic acid in the extract is equal to or higher than 80% by weight of the total triterpenic acids. the content of oleanolic acid and maslinic acid in the extract is equal to or higher than 85% by weight of the total triterpenic acids.

Hydroxytyrosol, also named 4-(2-Hydroxyethyl)benzene-1,2-diol, has the following formula:

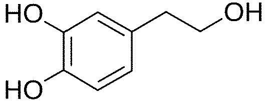

Several hydroxytyrosol extracts are commercially available and can be used for the purposes of the present invention.

Normally an olive extract in liquid form is selected, which usually is an aqueous extract. Such olive extract generally has a hydroxytyrosol content of at least 5% by weight, In a particular embodiment, the olive extract has a hydroxytyrosol content of at least 10% by weight, In another particular embodiment, the olive extract has a hydroxytyrosol content of at least 20% by weight, In another particular embodiment, the olive extract has a hydroxytyrosol content of at least 30% by weight, In another particular embodiment, the olive extract has a hydroxytyrosol content of at least 50% by weight. The extract may contain suitable carriers such as glycerine.

As a way of example, hydroxytyrosol can be generally obtained from olive mill wastewaters by (i) liquid-solid extraction of freeze-dried OMWW; (ii) liquid-liquid extraction of centrifuged OMWW; (iii) adsorption-desorption techniques using an adsorbent resin (Amberlite^{®} XAD) followed by gel permeation chromatograpy (Sephadex LH-20). Additional methodologies include supercritical CO₂ counter current column extraction, ultrafiltration and extraction with organic solvents. Hydroxytyrosol can also be obtained by chemical or biochemical synthesis using either Tyrosol or Oleuropein as staring material.

Specific preparation examples can be found for instance in Fernandez-Bolanos, Jose, Lopez, O., Fernandez-Bolanos, Juan, Rodriguez-Gutierrez, G., 2008. Hydroxytyrosol and Derivatives: Isolation, Synthesis, and Biological Properties. COC 12, 442-463. (see section 3: "isolation and purification of hydroxytryrosol); in US8066881B2 (see example 1), in US2010160690A1 (see examples) or in US2013309300 (see Examples 1-3).

A further example for obtaining an extract containing hydroxytyrosol in a high concentration is described in the patent application ES2186467A1 (see example 1). Basically, olives can be treated with a diluted solution of sodium hydroxide, the solution is generally removed after penetrating approximately 2/3 of the pulp, and the olives are covered with water. After several hours (10-15h), the wash water can be collected, stored anaerobically, and inoculated with lactic acid bacteria to lower the pH (<4.5) over around 12 months. The wash water may undergo ultrafiltration using for instance, a 4000 daltons molecular cutting tubular membranes. The ultrafiltered water can be treated with a nonionic resin (e.g. Amberlite XAD series). Adsorbed phenols can then be recovered with ethanol, which is later filtered off, the ethanol can be distilled, leaving a yellowish aqueous residue. The residue can be treated with activated carbon The aqueous extract can be frozen at -20°C and lyophilized to remove water. A solid residue with a high hydroxytyrosol concentration (>70%) can be obtained.

In another particular embodiment, in combination with any of the embodiments above or below, the product for use as defined above is that where the extract has an amount of one or more triterpenes in a range from 1 to 95 wt.%. In another particular embodiment, the product for use as defined above is that where the extract has an amount of one or more triterpenes in a range from 30 to 95 wt.%. In another particular embodiment, the product for use as defined above is that where the extract has an amount of one or more triterpenes in a range from 50 to 95 wt.%. In another particular embodiment, the product for use as defined above is that where the extract has an amount of one or more triterpenes in a range from 70 to 95 wt.%.

In another particular embodiment, in combination with any of the embodiments above or below, the product for use as defined above is that where the compound is a mixture of hydroxytyrosol and at least one triterpene and, where the extract comprises a mixture of hydroxytyrosol and at least one triterpene.

In another particular embodiment, in combination with any of the embodiments above or below, the product for use as defined above is that where the extract has an amount of hydroxytyrosol in a range from 0.5 to 95 wt.%. In another particular embodiment, the product for use as defined above is that where the extract has an amount of hydroxytyrosol in a range from 0.7 to 50 wt.%. In another particular embodiment, the product for use as defined above is that where the extract has an amount of hydroxytyrosol in a range from 1 to 20 wt.%. In another particular embodiment, the product for use as defined above is that where the extract has an amount of hydroxytyrosol in a range from 1 to 6 wt.%.

In a particular embodiment, in combination with any of the embodiments above or below, the product for use as defined above is that where the hydroxytyrosol and the at least one triterpene are in a ratio from 1:2 to 2:1 wt. %. In another particular embodiment, the product for use as defined above is that where the hydroxytyrosol and the at least one triterpene are in a ratio of 1:1 wt. %.

In a particular embodiment, in combination with any of the embodiments above or below, the product for use as defined above, wherein the compound is a mixture of hydroxytyrosol, maslinic acid, and oleanolic acid and, the extract comprises a mixture of hydroxytyrosol, maslinic acid, and oleanolic acid.

In a particular embodiment, in combination with any of the embodiments above or below, the product for use as defined above, wherein the administration is done between the days 13 to 21 of incubation. In another embodiment, the product for use as defined above, wherein the administration is done at day 17 of the incubation period.

The methods of the present invention are applicable to any species of bird, including but not limited to chickens, turkeys, ducks, geese, pigeons, quail, pheasants, and ostriches.

In a particular embodiment, in combination with any of the embodiments above or below, the product for use as defined above, wherein said bird is selected from the group consisting of chicken, turkey, duck, geese, pigeons, quail, pheasant, and ostrich.

In a particular embodiment, in combination with any of the embodiments above or below, the product for use as defined above is that where the maslinic acid and oleanolic are in a ratio from 1:10 to 10:1 wt. %. In another particular embodiment, the product for use as defined above is that where the maslinic acid and oleanolic are in a ratio from 1:5 to 5:1 wt. %. In another particular embodiment, the product for use as defined above is that where the maslinic acid and oleanolic are in a ratio from 1:2 to 2:1 wt. %. In another particular embodiment, the product for use as defined above is that where the maslinic acid and oleanolic are in a ratio of 1:1 wt. %. In another particular embodiment, the product for use as defined above is that where the maslinic acid and oleanolic are in a ratio of 11:3 wt. %. In another particular embodiment, the product for use as defined above is that where the maslinic acid and oleanolic are in a ratio of 4:1 wt. %.

In a particular embodiment, in combination with any of the embodiments above or below, the product for use as defined above is that where the compound for use further comprises reducing the mortality in poultry.

In another particular embodiment, in combination with any of the embodiments above or below, the product for use as defined above is that where the compound for use further comprises increasing the production of first class chicks in poultry.

In another particular embodiment, in combination with any of the embodiments above or below, the product for use as defined above is that where the compound for use further comprises increasing the hatchability in poultry.

In a particular embodiment, in combination with any of the embodiments above or below, the product for use as defined above, is that wherein the compounds are administered in the form of an injectable composition which comprises an effective amount of the compound or of the extract, optionally, together with acceptable excipients or carriers for injection, wherein the effective amount is an amount for enhancing conversion rate, reducing mortality, and increasing the first class chick production in poultry.

In a particular embodiment, in combination with any of the embodiments above or below, the product for use as defined above, wherein the effective amount of the compound or of the extract is an amount of 0.1 to 1 mg per egg. In a particular embodiment, the product for use as defined above, wherein the effective amount of the compound or of the extract is an amount of 0.3 to 0.7 mg per egg. In a particular embodiment, the product for use as defined above, wherein the effective amount of the compound or of the extract is an amount of 0.5 mg per egg.

In another particular embodiment, in combination with any of the embodiments above or below, the product for use as defined above is that where the product is particulated. In another particular embodiment, the particulated product are present in a crystalline or amorphous form suitable for *in ovo* administration. In another particular embodiment, the particle size of the product is in a range from 0.05 to 200 micrometers. In another particular embodiment, the particle size of the product is in a range from 1 to 100 micrometers. In another particular embodiment, the particle size of the product is in a range from 2 to 50 micrometers.

In the context of the present invention, the term *"Olea europaea"* refers to the species commonly known as the olive tree, which belongs to the Oleaceae family. *Olea europaea* is known for its various parts, including leaves, fruits (olives), and bark.

In a particular embodiment, the product for use as defined above is that where the extract is derived from *Olea europaea.* In another particular embodiment, the Extracts derived from Olea europaea, is a fruit (olives), extract, having special beneficial effects in poultry management.

In a particular embodiment, the extract from *Olea europaea* comprises triterpenic acids.

In another particular embodiment, the extract from *Olea europaea* is obtainable by process comprising the following steps: a) Centrifuging a lye reused from the production process of oxidized black olives; b) Acidifying the solid obtained; c) Drying the solid; d) Resuspending the solid in ethanol; e) Centrifuging; f) Adding water; g) Evaporating the organic solvent; and h) Lyophilizating the aqueous solution.

The centrifugation may be conducted at room temperature. The acidification is conducted with an acid such as phosphoric acid.

The maximum content of triterpenes in the extract is 95% in weight. In a particular embodiment, the extract has a triterpene content, in particular of maslinic acid and oleanolic acid equal to or higher than 70% in weight. In another particular embodiment, the extract has a triterpene content equal to or higher than 80 % in weight. In another particular embodiment, the extract has a triterpene content equal to or higher than 85 % in weight. In another particular embodiment, the extract has a triterpene content equal to or higher than 90 % in weight.

In a particular embodiment the product for use according to the invention is administered in the form of a sterile saline solution. In another particular embodiment, the concentration of the product in the saline solution is from 7 to 10 mg product/mL saline solution. In another particular embodiment, the concentration of the product in the saline solution is from 4 to 6 mg product/mL saline solution. When the product is an extract, the previous amounts correspond to the amount of product in the extract.

In a particular embodiment, the product for use as defined above, is that where the excipient is selected from the group consisting of buffers, stabilizers, surfactants, viscosity agents, antioxidants, antimicrobial agents, and preservatives.

In another particular embodiment, the product for use aa defined above, wherein the carrier is selected from the group consisting of starch and its derivatives, spray-gum, cellulose and its derivatives, proteins, inorganic salts, and emulsifiers.

Examples of appropriate starch and its derivatives include, but not limited to, starches, modified starch, waxy starch, arrowroot starch, carboxymethyl starch, wheat, maltodextrin, dextrin, cyclodextrins, corn syrup solids, pectin, trehalose, chitosan, and modified chitosan.

Examples of appropriate cellulose and its derivatives include, but is not limited to, cellulose, microcrystalline cellulose, hemicellulose, carboxymethyl cellulose, hydroxypropylmethyl cellulose phthalate, and methylcellulose.

Examples of appropriate gums include, but are not limited to, arabic/acacia gum, angum gum, guar gum, cashew gum, mesquite gum, xanthan gum, sodium alginate, and carrageenan.

Examples of appropriate proteins include, but are not limited to, caseins, wheat flour, soy protein, whey protein, pea protein, zein, and gelatin.

Examples of appropriate lipids include, but are not limited to, lecithin, oils, and fats.

Examples of appropriate inorganic salts include, but are not limited to, NaCl, calcium carbonate, SiOz.

In a particular embodiment, the product for use as defined above, is that where the injection is administered in the albumen of the egg. The term "albumen" refers to the clear, viscous liquid component of an egg.

Osmotic pressure is crucial when administering substances into living tissues or fluids. If the osmotic pressure of the nutrient composition is too high (hyperosmotic), it can cause dehydration or cellular damage to the embryo due to the movement of water out of the cells. Thus, in a particular embodiment, the product for use as defined above has an osmotic pressure equal to or lower than 400 milliosmoles per liter when administered.

In a particular embodiment, the product for use as defined above is that where the method for enhancing conversion rate in poultry further comprises combining the *in ovo* administration with administering a poultry feed composition during the poultry feed process.

Thus, a product which is either a compound selected from the group consisting of, at least a triterpene, and a mixture of at least a triterpene with hydroxytyrosol, or alternatively, an extract comprising at least a triterpene, or a mixture of at least a triterpene with hydroxytyrosol, for use in a method for enhancing conversion rate in poultry, wherein the method comprises administering the product *in ovo* between the days 7 to 21 of incubation, together with the use of a poultry feed composition in a poultry feed process is part of the invention.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### Examples

### Example 1. Preparation of extract containing triterpenes

Procedure for obtaining an extract containing triterpenic acids characterized by the use of olive dressing solutions, and comprising the following steps described in the patent application WO2010072874A1:
1 liter of lye reused from the production process of oxidized black olives, with an initial content of 13.9 g of maslinic acid and oleanolic acid, was used. It was centrifuged at 3500 rpm for 5 minutes at 20°C, and the resulting solid was acidified with 85% phosphoric acid until reaching a pH of 2.8. Subsequently, it was dried at 105°C for 20 hours and resuspended in 200 mL of ethanol for 10 minutes. The alcoholic solution was centrifuged at 3500 rpm for 5 minutes at 20°C, and 30 mL of water were added to the supernatant, which was subjected to vacuum evaporation to remove the organic solvent. The aqueous suspension was frozen at -30°C and lyophilized. 11.8 g of a whitish powder were obtained, with an 85% content of maslinic acid and oleanolic acid, where maslinic acid represented 70% of the total triterpenic acids.

### Example 2. Preparation of the injection to be administered

The active product is administered suspended in sterile saline solution. For this purpose: 1.88 g was weighed and suspended in a final volume of 50 mL of 0.7% saline solution with constant stirring at 500 rpm for 3 minutes. 5 mL was taken and diluted to a final volume of 20 mL of 0.7% saline solution, resulting in a final concentration of 9.4 mg/mL of extract providing 5 mg/mL of maslinic acid. 100 µL was injected into the albumen, delivering a total of 0.5 mg of maslinic acid per egg.

### Example 3. Assay in ovo on Ross 308 broilers injected with different compositions 960 eggs per group

a) A = injection of saline solution
b) B = injection of triterpene extract as defined in example 1.
d) C = injection of 1:1 mixture of b) and an hydroxytyrosol extract, which is a liquid olive fruit extract within ≥ 5%(w/w) hydroxytyrosol by HPLC, containing food grade vegetable glycerine as carrier (20-30%w/w)

For the following assays, the injection at day 17 of the incubation period. After hatching, broilers were followed in commercial conditions for 39 days (end of production cycle). Each treatment divided in 12 pens of 80 broilers. The broilers were the Ross 308 breed.

**Table 1. Death rates across different treatment modalities**

| **Deaths (%)** | **Saline** | **Triterpenes** |
|---|---|---|
| **Day** | **A** | **B** |
| 7 | 3.96% | 1.98% |
| 14 | 4.69% | 2.92% |
| 21 | 5.31% | 3.44% |
| 28 | 5.52% | 3.96% |
| 35 | 6.25% | 4.27% |
| 39 | 6.77% | 4.58% |

| | | |
|---|---|---|
| * Saline was used as the control sample. | | |

**Table 2. Feed Conversion Ratios (FCR) in kg of feed/kg gained across different treatment modalities**

| | **Saline** | **Triterpenes** | **Mixture 1:1 Hydroxytyrosol: Triterpenes** |
|---|---|---|---|
| | **A** | **B** | **C** |
| FCR(kg/kg) | 1.512 | 1.441 | 1.481 |

**Table 3. First class chicks rates across different treatment modalities**

| | **Saline** | **Triterpenes** |
|---|---|---|
| | **A** | **B** |
| 1st class chicks % | 86.7% | 89.5% |

**Table 4. Hatchability rates across different treatment modalities**

| | **Saline** | **Triterpenes** |
|---|---|---|
| | **A** | **B** |
| Hatchability % | 93.5% | 94.5% |

### Citation List

### Patent Literature

WO2021198235
WO2007096446A1
ES1286467A1
US8066881B2
US2010160690A1
US2013309300
ES2186467A1
WO2010072874A1

### Non Patent Literature

Habtamu Ayalew, Jing Wang, Shugeng Wu, Kai Qiu, Ayalsew Tekeste, Changchun Xu, Dessalegn Lamesgen, Sumei Cao, Guanghai Qi, Haijun Zhang, Biophysiology of in ovo administered bioactive substances to improve gastrointestinal tract development, mucosal immunity, and microbiota in broiler chicks, Poultry Science, Volume 102, Issue 12, 2023, doi: org/10.1016/j.psj.2023.103130.
Niknafs S, Meijer MMY, Khaskheli AA, Roura E. In ovo delivery of oregano essential oil activated xenobiotic detoxification and lipid metabolism at hatch in broiler chickens. Poult Sci. 2024 Feb;103(2):103321. doi: 10.1016/j.psj.2023.103321. Epub 2023 Nov 25. PMID: 38100943; PMCID: PMC10762474.
Dale MP et al.; "A systematic comparison of triterpenoid biosynthetic enzymes for the production of oleanolic acid in Saccharomyces cerevisiae". PLOS ONE. Vol. 15 (5): e0231980.
Fukushima EO et al.; (December 2011). "CYP716A subfamily members are multifunctional oxidases in triterpenoid biosynthesis". 2011, Plant & Cell Physiology. Vol. 52, pp. 2050-2061.
Fernandez-Bolanos, Jose, Lopez, O., Fernandez-Bolanos, Juan, Rodriguez-Gutierrez, G., 2008. Hydroxytyrosol and Derivatives: Isolation, Synthesis, and Biological Properties. COC 12, 442-463. https://doi.org/10.2174/138527208784083888

## Claims

1. A product which is either a compound selected from the group consisting of, at least a triterpene, and a mixture of at least a triterpene with hydroxytyrosol, or alternatively, an extract comprising at least a triterpene, or a mixture of at least a triterpene with hydroxytyrosol, for use in a method for enhancing conversion rate in poultry, wherein the method comprises administering the product *in ovo* between the days 7 to 21 of incubation.

2. The product for use according to claim 1, wherein the triterpene is selected from the group consisting of maslinic acid, oleanolic acid, and a mixture thereof.

3. The product for use according to claim 1-2, wherein the compound is a mixture of hydroxytyrosol and at least a triterpene and, the extract comprises a mixture of hydroxytyrosol and at least a triterpene.

4. The product for use according to any of the claims 1-3, wherein the hydroxytyrosol and the at least a triterpene are in a ratio from 1:2 to 2:1 wt. %.

5. The product for use according to claim 1-4, wherein the compound is a mixture of hydroxytyrosol, maslinic acid, and oleanolic acid and, the extract comprises a mixture of hydroxytyrosol, maslinic acid, and oleanolic acid.

6. The product for use according to any of the claims 1-5, wherein the maslinic acid and oleanolic are in a ratio from 1:10 to 10:1 wt. %.

7. The product for use according to any of the claims 1-6, wherein the administration is done at day 17 of the incubation period.

8. The product for use according to any of the claims 1-7, which further comprises reducing the mortality in poultry.

9. The product for use according to any of the claims 1-8, which further comprises increasing the production of first class chicks in poultry.

10. The product for use according to any of the claims 1-9, which further comprises increasing the hatchability in poultry.

11. The product for use according to any of the claims 1-10, wherein the compounds are administered in the form of an injectable composition which comprises either an effective amount of the compound or of the extract, optionally, together with acceptable excipients or carriers for injection, wherein the effective amount is an amount for enhancing conversion rate, and, optionally for reducing mortality, and/or increasing the first class chick production in poultry.

12. The product for use according to any of the claims 1-11, wherein the effective amount of the compound or of the compound in the extract to be administered is an amount in the range from 0.1 to 1 mg per egg.

13. The product for use according to any of the claims 1-12, wherein the extract is derived from *Olea europaea.*

14. The product for use according to any of the claims 1-13, wherein the injection is administered in the albumen of the egg.

15. The product for use according to any of the claims 1-14, wherein the method for enhancing conversion rate in poultry further comprises combining the *in ovo* administration with administering a poultry feed composition during the poultry feed process.
